(19) **Europäisches Patentamt / European Patent Office / Office européen des brevets**

(11) **EP 1 484 001 B1**

(12) **EUROPEAN PATENT SPECIFICATION**

(45) Date of publication and mention
of the grant of the patent:
**18.10.2017 Bulletin 2017/42**

(51) Int Cl.:
***A61B 1/00*** (2006.01)

(21) Application number: **03710356.1**

(86) International application number:
**PCT/JP2003/003070**

(22) Date of filing: **14.03.2003**

(87) International publication number:
**WO 2003/075752 (18.09.2003 Gazette 2003/38)**

(54) **ENDOSCOPE IMAGE PROCESSING APPARATUS**

ENDOSKOP-BILDBEARBEITUNGSGERÄT

APPAREIL DE TRAITEMENT D'IMAGE D'ENDOSCOPE

(84) Designated Contracting States:
**AT BE BG CH CY CZ DE DK EE ES FI FR GB GR HU IE IT LI LU MC NL PT RO SE SI SK TR**

(30) Priority: **14.03.2002 JP 2002070497**
**06.03.2003 JP 2003060153**

(43) Date of publication of application:
**08.12.2004 Bulletin 2004/50**

(73) Proprietor: **Olympus Corporation**
**Tokyo 192-8507 (JP)**

(72) Inventor: **KANEKO, Kazuma**
**Sagamihara-shi, Kanagawa 229-0003 (JP)**

(74) Representative: **von Hellfeld, Axel**
**Wuesthoff & Wuesthoff**
**Patentanwälte PartG mbB**
**Schweigerstrasse 2**
**81541 München (DE)**

(56) References cited:
| | |
|---|---|
| DE-A1- 10 101 064 | JP-A- 01 280 442 |
| JP-A- 03 280 920 | JP-A- 10 323 326 |
| JP-A- 2001 037 718 | JP-A- 2001 078 959 |
| JP-A- 2002 034 893 | US-A- 4 712 133 |
| US-A- 5 031 036 | US-A- 5 045 935 |
| US-A- 5 667 474 | US-A- 5 675 378 |
| US-A- 5 877 819 | US-A1- 2003 050 533 |

- WAGNIERES G A ET AL: "An endoscopic fluorescence imaging system for simultaneous visual examination and photodetection of cancers" REVIEW OF SCIENTIFIC INSTRUMENTS, AIP, MELVILLE, NY, US LNKD-DOI:10.1063/1.1147811, vol. 68, no. 1, 1 January 1997 (1997-01-01), pages 203-212, XP002552830 ISSN: 0034-6748

**Description**

Technical Field

The present invention relates to an endoscopic image processing system that processes an image signal produced by an endoscope.

Background Art

In recent years, endoscopes have been widely adopted in the field of medicine. Aside from endoscopes that perform ordinary image processing, endoscopes that perform image processing on the image data of a region to be examined in an object to be examined are available.

For example, Japanese Patent No. 2851116 has disclosed an electronic endoscope system in which control is extended in order to display both a raw image and a processed image on a monitor and to record the processed image and raw image.

According to the related art, it is judged whether an image displayed is a processed image or a raw image. If it is judged that the image is a processed image, the processed image is recorded in response to a directive of image recording. Thereafter, the raw image is automatically recorded. If it is judged that the image is a raw image, the raw image alone is recorded in response to the directive of image recording. Thus, the storage capacity of a recording medium is saved.

Document US5045935 discloses a n electronic endoscope system wherein an endoscopically-processed endoscopic image such as a color-enhanced endoscopic image is photographed with a simple operation. The electronic endoscope system comprises an endoscopic image producing unit for producing an endoscopic image signal of an object under medical examination as original endoscopic image data, an endoscopic image processing unit for processing the original endoscopic image data so as to obtain endoscopically-processed image data and a photographing unit for selectively photographing the original endoscopic image data and the endoscopically-processed image data. Document US4712133 discloses a n endoscopic apparatus comprising a push-button switch for supplying a freeze command signal to display an acquired video signal under frozen condition, a display for displaying a real image and/or a frozen image and a memory , wherein the apparatus enables the operator to judge whether the frozen image is adequate for recording it to the memory. However, according to the related art, if an image displayed is a processed image, two images of the processed image and raw image are always recorded. Even when the processed image alone is needed, the raw image is recorded indispensably. This leads to an increase in the number of recorded images. Moreover, after a processed image is recorded, the recorded image must be retrieved in order to see a raw image depicting the same scene. This is time-consuming.

The present invention attempts to break through the foregoing situation. An object of the present invention is to provide an endoscopic image processing system capable of recording a user-desired image according to a user's choice and preventing wasting of an image recording facility.

Another object of the present invention is to provide an endoscopic image processing system having maneuverability thereof improved so that after a processed image is recorded, a raw image depicting the same scene can be displayed.

Disclosure of Invention

An endoscopic image processing system for processing an image signal produced by imaging an intracavitary region of an object to be examined using an endoscope according to claim 1 is provided. The endoscopic image processing system may comprise:

an image producing means that produces a first endoscopic image, which can be displayed, by processing the image signal;

a processed image producing means that produces a second endoscopic image, which can be displayed, by processing the image signal or first endoscopic image;

a still image means that produces a first still image by freezing the image signal or first endoscopic image in response to a freeze signal transmitted with the handling of a freeze switch, and produces a second still image by freezing an image, which is represented by a predetermined domain within image data represented by the image signal, or the second endoscopic image in response thereto;

a memory means in which at least either of the first and second still images is temporarily stored;

an image recording means that records at least either of the first or second still images in response to a release signal transmitted with the handling of a release switch; and

a control means that controls the image recording means so that the second still image will be recorded responsively to the handling of the release switch, and consecutively displaying the first still image stored in the memory means.

When a user handles the release switch so as to direct exposure of the processed image produced by the processed image producing means, the second still image of the processed image whose exposure is directed by handling the release switch is recorded. Thereafter, the first still image depicting the same scene as the second still image does is displayed. Thus, recording or observation is performed based on a user's choice. This results in improved maneuverability.

Brief Description of the Drawings

Fig. 1 shows the overall configuration of an endoscope system including an exemplary embodiment of the present invention;
Fig. 2 is a block diagram showing the internal configuration of the endoscope system shown in Fig. 1;
Fig. 3 is a block diagram showing the configuration of an image processing block;
Fig. 4A to Fig. 4C show the examples of an ordinary image and a principal quasi image that are displayed on a monitor;
Fig. 5 is a flowchart describing actions to be performed for detecting the type of CCD and producing a mask signal;
Fig. 6A to Fig. 6D are explanatory diagrams showing screen images displayed when a release switch is handled in a normal viewing state;
Fig. 7A to Fig. 7F are explanatory diagrams showing screen images displayed when a release switch is handled in a quasi color image displaying state;
Fig. 8 is a flowchart describing the actions to be performed responsively to the handling of the release switch included in the present embodiment;
Fig. 9A to Fig. 9D are explanatory diagrams showing screen images displayed when the release switch is handled after the state in which a normal image is displayed in the form of a still image is changed to the quasi color image displaying state;
Fig. 10A to Fig. 10C are explanatory diagrams showing screen images that are variants of the screen images shown in Fig. 7A to Fig. 7F;
Fig. 11A to Fig. 11D are explanatory diagrams showing screen images that are variants of the screen images shown in Fig. 9A to Fig. 9D;
Fig. 12 is a block diagram showing the configuration of a light source unit needed to produce a normal image under visible light and also produce a fluorescence image;
Fig. 13 shows the structure of a spectrum limiting rotary filter included in the light source unit shown in Fig. 12;
Fig. 14 shows the structure of a RGB rotary filter;
Fig. 15 is an explanatory diagram concerning actions to be performed in a first mode; and
Fig. 16 is an explanatory diagram concerning actions to be performed in a second mode.

Best Mode for Carrying Out the Invention

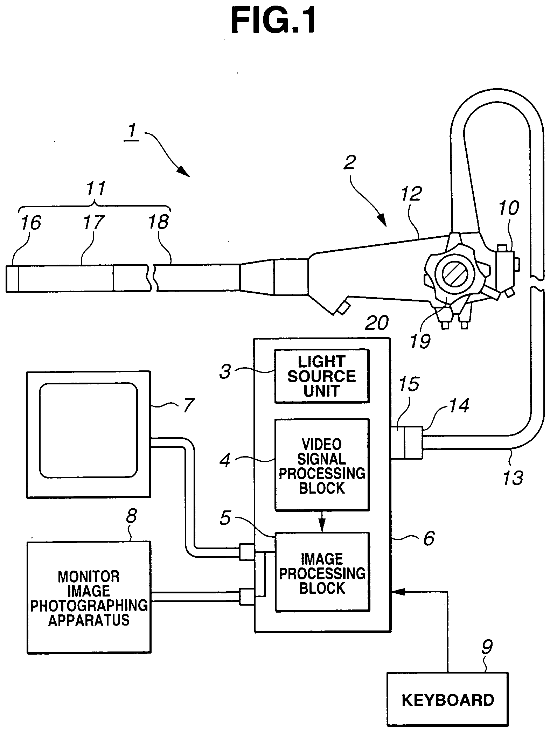

Referring to Fig. 1 to Fig. 16, an embodiment of the present invention will be described below.
As shown in Fig. 1, an endoscope system 1 having the present invention comprises: an electronic endoscope 2 including an image pickup means; a video processor 6 including a light source unit 3 that supplies illumination light to the electronic endoscope 2, a video signal processing block 4 that processes a video signal (image signal) sent from the image pickup means, and an image processing block 5 that performs image processing according to an output signal of the video signal processing block 4; a monitor 7 on which an image is displayed according to the image signal sent from the video processor 6; a monitor image photographing apparatus 8 that photographs a monitor image (endoscopic image) displayed on the monitor 7; and a keyboard 9 connected to the video processor 7 and used to transmit a directive signal of directing that image processing should be enabled or disabled or to enter patient data.
The electronic endoscope 2 has an elongated and, for example, movable insertion unit 11. A large-diameter operation unit 12 is joined to the rear end of the insertion unit 11. A flexible universal cord 13 is extended from the lateral side of the rear part of the operation unit 12. A connector 14 fixed to the end of the universal cord 13 is freely detachably attached to a connector receptacle 15 of the video processor 6.
The insertion unit 11 has, from the distal end thereof,: a hard distal section 16; a bending section 17 that can be freely bent and adjoins the rear end of the distal section 16; and an elongated flexible section 18 that has flexibility. Moreover, the bending section 17 can be bent in a rightward, leftward, upward, or downward direction by turning an angulation knob 19 included in the operation unit 12. Moreover, the operation unit 12 has an insertion port 20 that communicates with a treatment instrument channel running through the insertion unit 11.

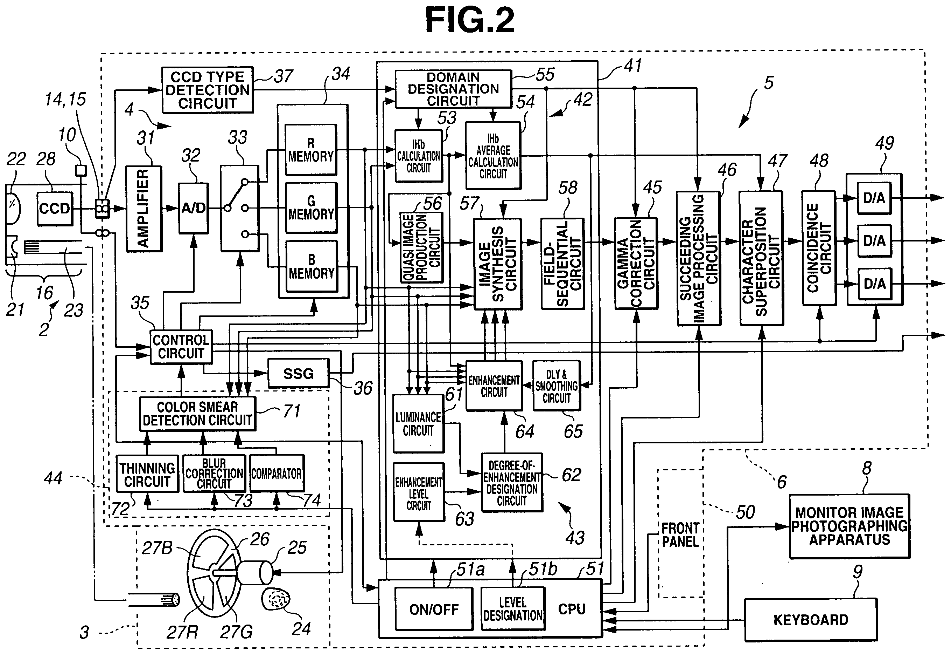

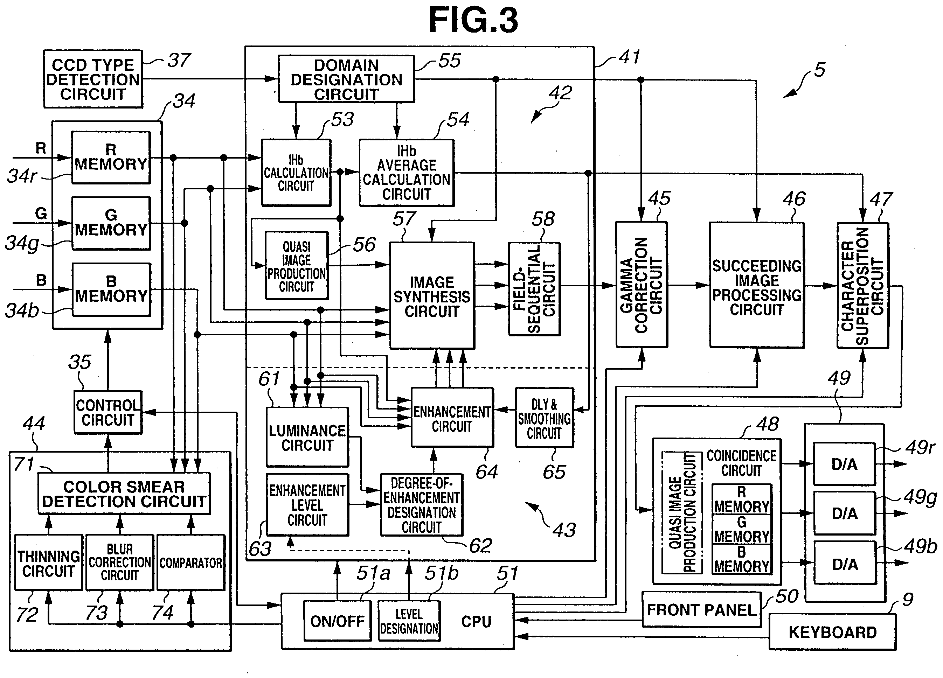

Moreover, scope switches 10 including a freeze switch used to direct freeze and a release switch used to direct exposure are located on the top of the operation unit 12 of the electronic endoscope 2. When the scope switch 10 is handled in order to, for example, direct freeze, the directive signal is transferred to a control circuit 35 (see Fig. 2) incorporated in the video processor 6. The control circuit 35 controls a memory unit 34 (red, green, and blue memories 34r, 34g, and 34b included in the memory unit 34) (see Fig. 3) so that a frozen image will be displayed.

When the keyboard 9 or a freeze switch included in a front panel 50 (see Fig. 2) of the video processor 6 is handled in order to direct freeze, the directive signal is transmitted to the control circuit 35 via a CPU 51 (see Fig. 2). The control circuit 35 extends control appropriately.

Moreover, when a release switch is handled in order to direct exposure, a release signal is transmitted to the monitor image photographing apparatus 8 with a frozen image displayed. The frozen image is then photographed.

As shown in Fig. 2, an illumination lens 21 and an objective optical system 22 are locked in an illumination window and an observation window respectively formed in the distal section 16. A light guide 23 formed with a fiber bundle is disposed behind the illumination lens 21. The light guide 23 runs through each of the insertion unit 11, operation unit 12, and universal cord 13, and is then coupled to the connector 14.

When the connector 14 is attached to the video processor 6, illumination light emanating from the light source unit 3 incorporated in the video processor 6 falls on the incident end of the light guide 23.

The light source unit 3 includes a lamp 24 and a rotary filter 26 that is located on the path of illumination light emanating from the lamp 24 and rotated by a motor 25.

The lamp 24 emits visible light. The rotary filter 26 has color transmission filters 27R, 27R, and 27B, which transmit light rays having mutually different regions of wavelengths, arranged circumferentially. The color transmission filters 27R, 27G, and 27B of the rotary filter 26 are designed to exert the characteristics of passing light rays that belong to the respective regions of wavelengths determining red, green, or blue.

Light emanating from the lamp 24 has light rays, which belong the respective regions of wavelengths, time-sequentially separated from one another by the rotary filter 26, and falls on the incident end of the light guide 23. The illumination light is introduced to the distal section 16 by the light guide 23, and passes through the illumination lens 21 locked in the illumination window on the distal plane. Consequently, an object such as a region to be examined is illuminated with red, green, and blue field-sequential illumination light rays.

On the other hand, a solid-state image pickup device, for example, a charge-coupled device (CCD) 28 is located at the position of the image formation of the objective optical system 22. An object image of the object illuminated with field-sequential illumination light is converged on the photoelectric conversion surface of the CCD 28 by the objective optical system 22, and converted into an electric signal by the CCD 28. An image signal (image pickup signal) resulting from the photoelectric conversion performed by the CCD 28 is transferred to the video signal processing block 4, and applied to an amplifier 31 that amplifies the electric image signal up to a voltage falling within a predetermined range (for example, 0 to 1 volt).

An output signal of the amplifier 31 is converted into a digital signal by an A/D converter 32, and transferred to a selector 33 that has one input terminal and three output terminals. Red, green, and blue signals sent time-sequentially are separated from one another by the selector 33, and transferred to the memory unit 34.

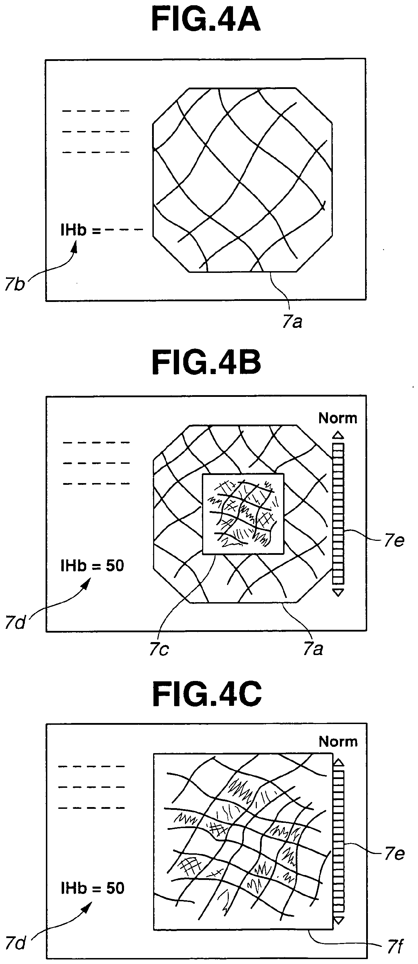

The separated red, green, and blue color signals are stored in the associated red, green, and blue memories 34r, 34g, and 34b (included in the memory unit 34) (see Fig. 3 and Fig. 4) (34r, 34g, and 34b are not written in Fig. 2).

Analog-to-digital conversion performed by the A/D converter 32 and storing (writing) or reading of color signals in or from the red, green, and blue memories 34r, 34g, and 34b of the memory unit 34 are controlled by the control circuit 35. Moreover, the control circuit 35 transmits a reference signal to a sync signal generation circuit (SSG in Fig. 2) 36. The sync signal generation circuit 36 generates a sync signal synchronous with the reference signal. When writing in the red, green, and blue memories 34r, 34g, and 34b is inhibited, a state in which a still image is displayed is attained (the same applies to a case where writing in red, green, and blue memories included in a coincidence circuit 48 to be described later is inhibited).

Moreover, according to the present invention, the signal processing can be adapted to signals produced by the electronic endoscope 2 whose CCD 28 offers a different number of pixels. Consequently, the connector receptacle 15 includes a CCD type detection circuit 37 that detects the type of CCD 28 incorporated in the electronic endoscope 2 actually connected to the video processor 6, which determines the number of pixels, by checking the number of pins included in the connector 14.

The means for detecting the type of CCD 28 that determines the number of pixels or the like is not limited to the circuit that detects the number of pins of the connector 14 associated with the number of pixels offered by the CCD 28. Alternatively, a circuit that applies a CCD driving signal to the CCD 28 and detects the number of pixels (number of horizontal image dots and number of vertical image dots) from the number of waveform peaks exhibited by an output signal of the CCD 28.

Red, green, and blue color signals read from the memories 34r, 34g, and 34b respectively are transferred to an IHb processing block 41 that is included in the image processing block 5. The IHb processing block 41 calculates a value (IHb) correlating with an amount of hemoglobin serving as hematic information.

According to the present embodiment, the IHb processing block 41 includes: an IHb processing circuit 42 that calculates an IHb value from each pixel, calculates an average of IHb values, or produces a quasi color image, which is displayed, on the basis of the IHb values calculated from pixels; and a color enhancement circuit 43 that enhances a color.

Moreover, the present embodiment includes a color smear detection block 44 that when a quasi color image based on IHb values is displayed as a frozen image, detects color smear so that the quasi color image can be displayed with little color smear.

A field-sequential signal sent from the IHb processing block 41 undergoes gamma correction performed by a gamma correction circuit 45, and further undergoes structure enhancement performed by a succeeding image processing circuit 46. Thereafter, a character superposition circuit 47 superposes patient data or a calculated average of IHb values on an image. Thereafter, the coincidence circuit 48 converts the field-sequential signal into a coincident signal.

The coincidence circuit 48 has, as shown in Fig. 3, red, green, and blue memories. The coincidence circuit 48 temporarily writes the red, green, and blue signal components of the field-sequential signal in the red, green, and blue memories, simultaneously reads stored red, green, and blue signal components, and thus transmits red, green, and blue signals that are coincident with one another.

The red, green, and blue signals that are coincident with one another are transferred to three D/A converters 49r, 49g, and 49b (see Fig. 3) (49r, 49g, and 49b are not written in Fig. 2) included in the D/A converter 49. The red, green, and blue signals are converted into analog red, green, and blue signals, and transferred to each of the monitor 7 and monitor image photographing apparatus 8. Incidentally, writing or reading in or from the red, green, and blue memories included in the coincidence circuit 48, and digital-to-analog conversion by the D/A converter 49 are controlled by the control circuit 35.

The monitor image photographing apparatus 8 includes a monitor on which, similarly to the monitor 7, an image is displayed and which is not shown, and a photographing device (more particularly, a camera) that records an image displayed on the monitor through photography.

A user displays a normal image (raw image), which is produced under illumination light of normal visible light, on the monitor 7. Moreover, the user handles the switch on the front panel 50 of the video processor 6 or the keyboard 9 so as to direct display of an IHb image. The directive signal is transferred to the CPU 51. The CPU 51 controls the IHb processing block 41 or the like according to the directive signal.

Next, the configuration of the IHb processing block 41 will be described below. As shown in Fig. 2 and Fig. 3, a red signal read from the red memory 34r and a green signal read from the green memory 34g are transferred to an IHb calculation circuit 53 included in the IHb processing block 41. IHb values are then calculated. The IHb values are transmitted to an IHb average calculation circuit 54 that averages the IHb values.

Moreover, the information of the type of CCD detected by the CCD type detection circuit 37 is transferred to a domain designation circuit 55. The domain designation circuit 55 designates a quasi image display domain according to the information of the type of CCD so that a quasi image will be displayed in an appropriate size.

Moreover, the information of the domain designated by the domain designation circuit 55 is transmitted to each of the IHb calculation circuit 53 and IHb average calculation circuit 54. An IHb value is calculated from each of pixels constituting the domain.

The IHb calculation circuit 53 calculates an IHb value from each pixel by performing the arithmetic operation expressed with the formula (1) below.

$$\mathtt{IHb\ =\ 32\ \times\ Log^2(R/G)} \qquad\qquad (1)$$

where R denotes red image data and G denotes green image data.

It is easy to implement the formula (1) in a circuit. For example, a divider that is not shown is used to perform an arithmetic operation on red image data and green image data. A logarithmic conversion table that is not shown and formed with a ROM or the like is used to convert the result of the arithmetic operation. Alternatively, a CPU or the like may be used to perform the arithmetic operation expressed with the formula (1).

IHb values calculated by the IHb calculation circuit 53 are transmitted to the IHb average calculation circuit 54. The IHb average calculation circuit 54 averages some of the received IHb values that are calculated from the domain defined by the domain designation circuit 55, and thus calculates an IHb average.

The IHb values calculated by the IHb calculation circuit 53 are also transferred to a quasi image production circuit 56. The quasi image production circuit 56 produces a quasi image, which is displayed in quasi colors, from the IHb values, and transmits the quasi image to an image synthesis circuit 57 that synthesizes images.

The image synthesis circuit 57 receives the quasi image data produced by the quasi image production circuit 56 and red, green, and blue image data items read from the red, green, and blue memories 34r, 34g, and 34b respectively. The image synthesis circuit 57 synthesizes the image data items according to a mask signal sent from the domain designation circuit 55. The synthetic image data is transmitted to a field-sequential circuit 58 that converts received data into a field-sequential signal.

More particularly, the image synthesis circuit 57 transmits red, green, and blue image data items that represent a raw image during a period during which the mask signal assumes a logical 0, and transmits the quasi image data during a period during which the mask signal assumes a logical 1. The image synthesis circuit 57 thus produces synthetic image data and transmits it to the succeeding field-sequential circuit 58.

The field-sequential circuit 58 field-sequentially transmits the red, green, and blue components of the synthetic image data. In other words, image data items of red, green, and blue components are field-sequentially transmitted to the gamma correction circuit 45.

According to the present embodiment, the information of the domain designated by the domain designation circuit 57 (more particularly, a mask signal) is transmitted to each of the gamma correction circuit 45 and succeeding image processing circuit 46. The actions of the gamma correction circuit 45 and succeeding image processing circuit 46 are disabled responsively to the enablement of quasi color display. Gamma correction and structure enhancement may be performed on raw image data, which surrounds a predetermined domain, via the CPU 51 according to a user's choice made at the keyboard 9.

Moreover, an IHb average calculated by the IHb average calculation circuit 54 is transmitted to the character superposition circuit 47. Consequently, the calculated IHb average can be displayed on the monitor screen. Even in this case, a user can select the display or non-display via the CPU 51.

Moreover, the red, green, and blue image data items read from the red, green, and blue memories 34r, 34g, and 34b are transferred to a luminance circuit 61 included in the color enhancement circuit 43. A luminance signal is then produced and transferred to a degree-of-enhancement designation circuit 62 that designates a degree of enhancement.

The degree-of-enhancement designation circuit 62 receives an enhancement level determined by an enhancement level circuit 63 that determines an enhancement level. The degree-of-enhancement designation circuit 62 designates a degree of enhancement from the signal level of the luminance signal and the enhancement level alike, and transmits a signal representing the degree of enhancement to an enhancement circuit 64.

The enhancement circuit 64 receives the red, green, and blue image data items read from the red, green, and blue memories 34r, 34g, and 34b respectively and the IHb values calculated by the IHb calculation circuit 53. Moreover, the enhancement circuit 64 receives the IHb average calculated by the IHb average calculation circuit 54 via a delay and smoothing circuit (DLY & smoothing circuit in Fig. 2 and Fig. 3) 65.

The enhancement circuit 64 performs color enhancement and transmits the resultant red, green, and blue image data items to the image synthesis circuit 57. When color enhancement is enabled, the image synthesis circuit 57 may synthesize quasi color data with color-enhanced image data but not with raw image data. Needless to say, when quasi color display is disabled, only image data that has colors enhanced and that is transmitted from the enhancement circuit 64 may be transmitted. Otherwise, raw image data and color-enhanced image data may be synthesized instead of raw image data and quasi color data, and the resultant synthetic image data may be transmitted.

In this case, the color-enhanced image data refers to color-enhanced red, green, and blue image data items produced based on the IHb values, IHb average, luminance level, and enhancement level.

Moreover, in case color-enhanced image data is synthesized with raw image data but not with quasi color image data, the color-enhanced image data is synthesized with the domain of the raw image data with which the quasi color data is supposed to be synthesized. When full-screen display rather than partial display is designated, an image represented by the color-enhanced image data is fully displayed on the entire screen.

A user displays a normal image (raw image), which is produced under normal visible illumination light, on the monitor 7 or handles the directive switch on the front panel 50 of the video processor 6 or the keyboard 9 so as to direct display of an IHb image. The directive signal is then transferred to the CPU 51. The CPU 51 controls the IHb processing block 41 in response to the directive signal.

In this case, the user handles the switch on the front panel 50 or the keyboard 9 so as to direct the enablement or disablement of the display of an IHb image (quasi image) or the enablement or disablement of color enhancement. The CPU 51 controls the enablement or disablement (on or off) 51a in response to the directive. When designation of an enhancement level is directed, the CPU 51 controls level designation 51b which the enhancement level circuit 63 performs to designate an enhancement level.

Moreover, the red, green, and blue image data items read from the red, green, and blue memories 34r, 34g, and 34b are transferred to a color smear detection circuit 71 included in the color smear detection block 44. A degree of correlation to which the red, green, and blue image data items are correlated with one another is detected in order to detect a degree of color smear.

When freeze of an image is directed, an image suffering the least color smear is detected. A signal is transmitted to the control circuit 35 so that the image having the least color smear detected during a field will be displayed. The control circuit 35 inhibits writing in the red, green, and blue memories 34r, 34g, and 34b respectively included in the memory unit 34. The control circuit 35 then extends control so that still images will be displayed as an image to be displayed on the monitor 7 serving as a display means and as an image to be displayed on the monitor of the monitor image photographing apparatus 8.

As a method of detecting a degree of color smear in an image to be frozen, one of three color smear detection methods to be described below is selected or a combination thereof is adopted in order to detect an image suffering the least color smear.

To be more specific, the color smear detection circuit 71 uses a thinning circuit 72, a blur correction circuit 73, or a comparator 74 to detect color smear.

When a large number of images must be checked to see if they suffer color smear, the thinning circuit 72 is used to thin out the number of time-sequential images. The resultant number of images is checked to see if they suffer color smear. Consequently, a wide range of images can be dealt with.

Using the comparator 74, two methods are conceivable. One of the methods employs a multi-stage comparator so that changes in a plurality of field images concerned can be compared with one another at a time in order to search an image suffering the least color smear. (This method has the demerits that the scale of a circuit becomes large and the number of images that can be dealt with is limited, but has the merit that since fast processing is possible, an image suffering the least color smear can be searched for a short period of time.) The other method is such that two of a plurality of images are compared with each other in order to search an image suffering the least color smear. (This method has the merits that the scale of a circuit is small and the number of images that can be dealt with is larger, but has the demerit that since fast processing is impossible, it takes much time to search an image suffering the least color smear).

According to an image construction method, the display positions of red, green, and blue images constituting an image that suffers the least color smear and that is searched as mentioned above are two-dimensionally shifted in units of several pixels. Thus, an image whose color smear is further minimized is constructed. This method, that is, blur correction is implemented in the blur correction circuit 73.

According to the above method, since red, green, and blue images are displaced, the edges of the images are invalidated. This results in a smaller view image. Nevertheless, the method has the merit of reducing color smear.

A menu may be used to select any of the foregoing methods or to designate a combination thereof in advance. Otherwise, the methods may be switched based on settings. For example, one of the methods or a combination thereof is specified in an item of pre-freeze in a freeze menu that is not shown.

In the pre-freeze item, a numerical value to be entered as, for example, a freeze level corresponds to the number of images that can be dealt with. When the numerical value is, for example, 8 indicating the highest level, since the numerical value is equal to or larger than the limiting number of images that can be dealt with through multi-stage comparison, the method of comparing two of images with each other is adopted. If the level is lower than level 8, since the numerical value is smaller than the limiting number of images that can be dealt with through multi-stage comparison, color smear is detected through multi-stage comparison.

Moreover, if On is specified in an item of blur correction in the freeze menu screen image, blur correction is performed on an image that suffers the least color smear and that is searched according to any of the aforesaid methods. Consequently, an image whose color smear is further minimized can be constructed.

According to the present embodiment, the CCD type detection circuit 37 detects the type of CCD 28 actually incorporated in the electronic endoscope 2 which determines the number of pixels. A domain of pixels to be used to display a quasi image is designated relative to all the number of pixels determined with the detected type. Consequently, even when a different type of electronic endoscope 2 is connected for use, a quasi image is automatically displayed in an appropriate size.

To be more specific, in a normal operational state, endoscopic images are, as shown in Fig. 4A, displayed in the form of a motion picture in an octagonal endoscopic image display area 7a on the display surface of the monitor 7. Moreover, patient data as well as an indication signifying that an IHb average is ready for display is displayed.

Specifically, IHb=--- (7b in Fig. 4A) is displayed, signifying that an IHb average is ready for display. The indication signifying that an IHb average is ready for display may be displayed or not according to a user's choice. The IHb average provides information that helps distinguish a lesion from a sound (normal) region.

When display of a quasi color image is enabled, a quasi color image is, as shown in Fig. 4B, displayed in a mask area 7c in the center of the endoscopic image display area 7a. In this case, an IHb average (7d in Fig. 4B) can be displayed instead of the indication signifying that an IHb average is ready for display.

A quasi color image (that is a processed image or a second endoscopic image) is displayed in the mask area 7c in which the center of an endoscopic image depicting an object of observation, that is, a region of interest is displayed. A raw image (that is a first endoscopic image) is displayed around the mask area.

In this case, a color bar 7e indicating the range of tones employed in a quasi color image is displayed by the right side of the endoscopic image display area 7a. In the example, a normal (Norm) range of tones is indicated.

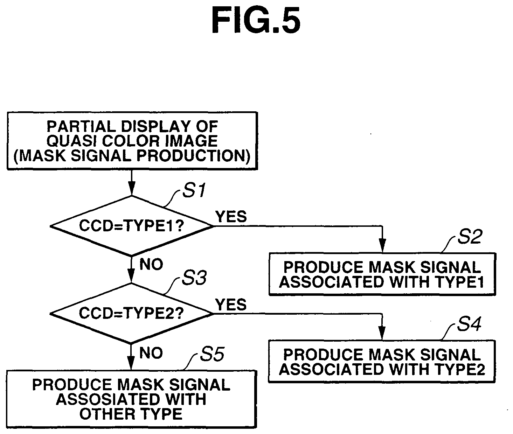

According to the present embodiment, a quasi color image is, as shown in Fig. 4B, displayed in the mask area 7c in the center of the endoscopic image display area 7a (that is, a quasi color image is partly displayed). In this case, the domain designation circuit 55 generates a mask signal according to a sequence described in Fig. 5. Even when the CCD 28 employed offers a different number of pixels, a quasi color image can be partly displayed in an appropriate size.

Specifically, when a quasi color image is partly displayed, the domain designation circuit 53 determines from a detection signal sent from the CCD type detection circuit 37 at step S1 described in Fig. 5 whether the actually employed CCD 28 is of type 1. If it is determined that the CCD 28 is of type 1, a mask signal associated with type 1 is generated at step S2, and transmitted to the image synthesis circuit 57.

The mask signal associated with type 1 is a binary signal that assumes logical 1 at the timing of partly displaying a quasi color image, and that assumes logical 0 during the other period. The image synthesis circuit 57 performs image synthesis to partly fit a quasi color image in a raw image according to the mask signal, and transmits a resultant image to the succeeding stage. Consequently, a quasi color image is, as shown in Fig. 4B, partly displayed on the display surface of the monitor 7.

On the other hand, if it is determined that the CCD 28 employed is not of type 1, control is passed to step S3. It is then determined whether the CCD 28 is of type 2. If it is determined that the CCD 28 is of type 2, the domain designation circuit 53 generates a mask signal associated with type 2 at step S4, and transmits the mask signal to the image synthesis circuit 57. Even in this case, a quasi color image is, nearly the same as shown in Fig. 4B, partly displayed.

On the other hand, if the condition presented at step S3 is not met, control is passed to step S5. A mask signal associated with any other type of the CCD 28 is generated. Even when a plurality of types of CCD 28 can be employed, a quasi color image is partly displayed in a size appropriate for the number of pixels offered by the actually employed CCD 28 (unlike the related art, without the necessity of designating a domain of pixels from the number of pixels offered by the CCD 28).

A description will be made by taking a concrete example. For example, the CCD 28 of type 1 offers the number of pixels corresponding to a product of 400 by 400. In this case, a binary mask signal is generated so that the mask signal will assume logical 1 relative to the domain of pixels corresponding to a square matrix of 200 pixels in the center of the square matrix of 400 pixels (that is, a quarter size). The mask signal will assume logical 0 during a period during which the other pixels are dealt with. Moreover, the CCD 28 of type 2 offers the number of pixels corresponding to a product of 800 by 600. In this case, a binary mask signal is generated so that the mask signal will assume logical 1 relative to the domain of pixels corresponding to a square matrix of 400 by 300 (a quarter size) in the center of the square matrix of 800 by 600. The mask signal will assume logical 0 during a period during which the other pixels are dealt with.

Consequently, whichever of the CCDs of type 1 and type 2 is employed, a quasi color image is partly displayed in the center of a raw image in a quarter display size.

According to the present embodiment, not only the display mode in which a quasi color image is partly displayed as shown in Fig. 4B is supported but also a display mode in which an entire quasi color display (7f) is fully displayed as shown in Fig. 4C is supported. Depending on a user's choice, if (partly) displaying a quasi color image in the center is designated (enabled), partial display is performed as shown in Fig. 4B. If displaying a quasi color image in the center is not designated, full display is performed as shown in Fig. 4C. Incidentally, the entire quasi color display 7f may be fully displayed in the same octagonal area as the octagonal endoscopic image display area shown in Fig. 4A.

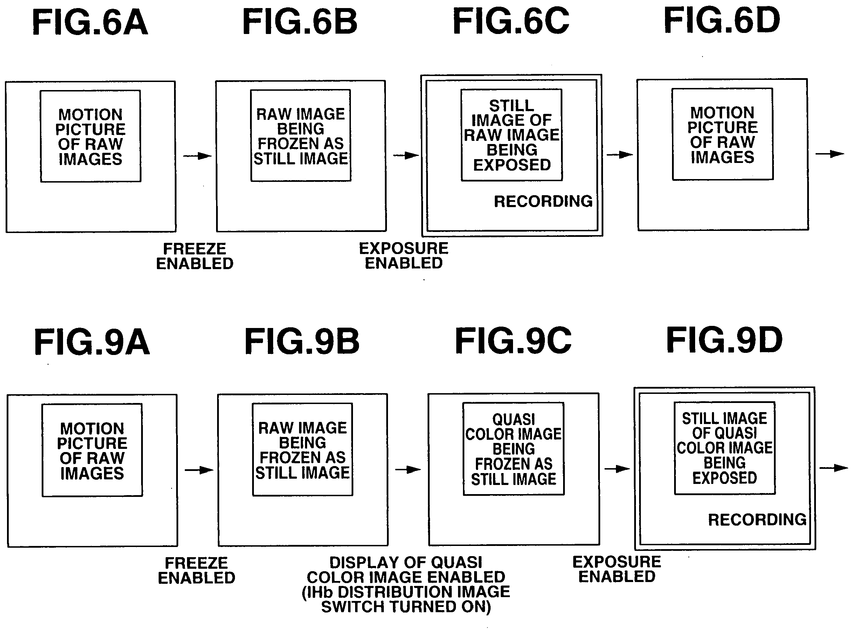

According to the present embodiment, if a release switch is handled in normal motion picture viewing mode, processing is performed to cause changes shown in Fig. 6A to Fig. 6D.

Specifically, when raw images are, as shown in Fig. 6A, displayed in the form of a motion picture, if a freeze switch is handled, a raw image is, as shown in Fig. 6B, frozen and displayed as a still image.

When a release switch is handled, the still image shown in Fig. 6B is recorded as shown in Fig. 6C. When the recording is completed, display of raw images in the form of a motion picture is resumed as shown in Fig. 6D.

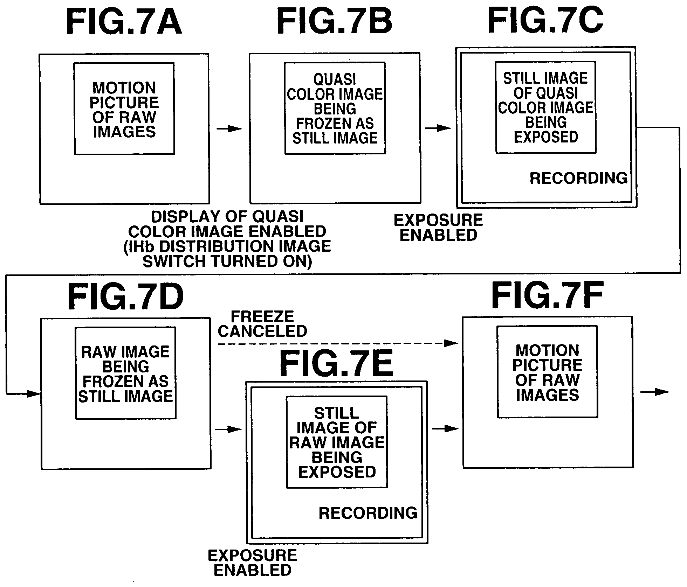

According to the present embodiment, if display of a quasi color is enabled in the normal motion picture viewing mode, a raw image is frozen and a quasi color is displayed as shown in Fig. 7B. In this case, if the release switch is handled, after recording is performed, a raw image corresponding to a quasi color is frozen and displayed as a still image. A user can therefore view the raw image corresponding to the recorded quasi color. If necessary, the still image can be recorded.

In other words, when a directive that a quasi color should be recorded as processed image of a raw image is given, the processed image is recorded. Thereafter, a still image of the raw image corresponding to the processed image is automatically displayed. If a user wants to record the still image, the still image is recorded. If the user does not want to record the still image but merely wants to view it, the user can merely view the still image. Thus, maneuverability has improved so as to cope with any user's choice.

As shown in Fig. 2, the CPU 51 monitors the handling of the keyboard 9 or the like for directing exposure of a raw image other than a quasi color as well as the handling of the release switch therefor with a quasi color displayed. If exposure of a raw image is directed, a release signal is transmitted to the monitor image photographing apparatus 8 so that photography will be performed.

Moreover, when photography performed in response to the release signal is completed, the CPU 51 receives a photography end signal from the monitor image photographing apparatus 8. After photography of a quasi color display is completed, the CPU 51 transmits a control signal to the control circuit 35 so that the control circuit 35 will display a still image of the quasi color that is a processed image.

After the CPU 51 receives a photography end signal from the monitor image photographing apparatus 8, the CPU 51 displays a still image of a corresponding raw image. The present invention is not limited to this mode. Alternatively, after a release signal is transmitted to the monitor image photographing apparatus 8, when the time that is long enough to perform photography has elapsed, the CPU 51 may display a still image of a corresponding raw image.

A quasi color shown in Fig. 7B or any other drawing may be partly displayed in the center of a raw image as shown in Fig. 4B or fully displayed as shown in Fig. 4C. Moreover, when the freeze switch is handled, an image changes to a state indicated in a drawing shown by the right side of the drawing showing the image. For example, a partly displayed image changes to a state, which is indicated in a drawing shown by the right side of the drawing showing the image, as it is. Likewise, a fully display image changes to a state, which is indicated in a drawing shown by the right side of the drawing showing the image, as it is.

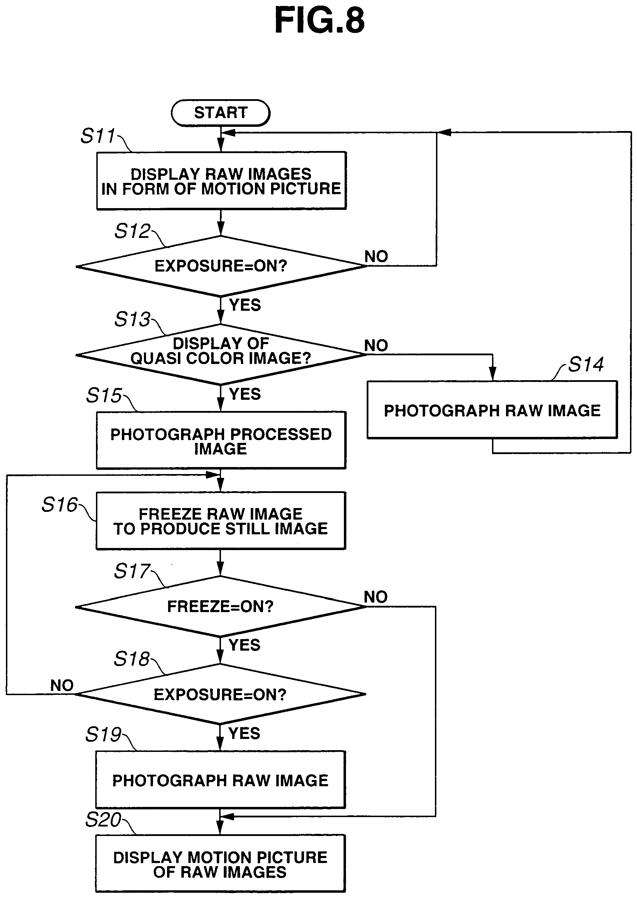

Next, referring to Fig. 8, a description will be made of actions to be performed responsively to the handling of the release switch.

As described in Fig. 8, when the power supply of the endoscope system 1 is turned on, raw images are displayed on the monitor 7 in the form of a motion picture at step S11. At step S12, the CPU 51 determines whether the release switch has been handled, that is, whether exposure of an image has been enabled.

If the release switch has not been handled, control is returned to step S11. If the release switch has been handled, control is passed to step S13. At step S13, it is determined whether display of a quasi color has been enabled. If display of a quasi color has not been enabled, a raw image is frozen at step S14, and then photographed. Thereafter, raw images are displayed in the form of a motion picture.

This case is equivalent to the normal motion picture viewing mode described in conjunction with Fig. 6A to Fig. 6D. Referring to Fig. 6A to Fig. 6D, a description has been made on the assumption that the release switch is handled after the freeze switch is handled. When the release switch is handled before the freeze switch is operated, an image is frozen as it is at step S14. Thereafter, exposure (photography) is carried out. Consequently, the same actions as those indicated in Fig. 6A to Fig. 6D are performed.

If it is found at step S13 that display of a quasi color has been enabled, control is passed to step S15. A processed image, that is, a quasi color image is photographed.

In this case, when display of a quasi color is enabled (IHb distribution image switch is turned on) in the state shown in Fig. 7A, a quasi color (image) is frozen and displayed as a still image as shown in Fig. 7B. Thereafter, when the release switch is handled (exposure is enabled), a still image of a quasi color (image) shown in Fig. 7B is, as shown in Fig. 7C, exposed or photographed and thus recorded.

When the photography is completed, a still image of a raw image is displayed at step S16. In other words, a raw image corresponding to the photographed quasi color (image) is, as shown in Fig. 7D, frozen and displayed as a still image.

Consequently, when a user wants to photograph a raw image corresponding to a quasi color image for the purpose of comparison, the user should enable exposure. If photography is unnecessary, freeze should canceled. The CPU 51 determines at step S17 whether freeze has been enabled or designated. If freeze has not been enabled (that is, freeze has been canceled), control is passed to step S20. A motion picture composed of raw images is displayed, that is, the state shown in Fig. 7F is attained.

On the other hand, when freeze is enabled or designated, it is determined at step S18 whether exposure has been enabled. If exposure has not been enabled, control is returned to step S16. The state in which a still image of a raw image is displayed is maintained. When exposure has been enabled, a raw image displayed as a still image is, as shown in Fig. 7E, photographed and thus recorded. After the recording is completed, control is passed to step S20. Raw images are then displayed in the form of a motion picture.

According to the present invention that performs the foregoing actions, when a quasi color image is photographed and thus recorded, a raw image corresponding to the quasi color image is frozen and displayed afterward. A user can readily photograph and record a raw image without labor. The user can designate mere viewing of images not including photography. Thus, a system with excellent maneuverability can be provided.

According to the related art, when a processed image is recorded, a corresponding raw image is also recorded inevitably. According to the present embodiment, if a user judges that recording is unnecessary, recording is not performed. Recording or the like can be designated based on an actual use situation. Moreover, when a processed image is photographed, a corresponding raw image is displayed in the form of a still image. The corresponding raw image can therefore be viewed without labor. Moreover, whether an image should be recorded or merely viewed can be designated easily.

Referring to the flowchart of Fig. 8, actions to be performed for photography in the states shown in Fig. 6A to Fig. 6D or Fig. 7A to Fig. 7F have been described. According to the present embodiment, if an image is, as shown in Fig. 9B, displayed in the form of a still image, after display of a quasi color is enabled, exposure may be performed. Actions to be performed after the exposure is completed are identical to those performed in the states shown in Fig. 7C and succeeding images.

Specifically, Fig. 9A and Fig. 9B show, similarly to Fig. 6A and Fig. 6B, a state in which a motion picture is displayed and a state in which the freeze switch is handled. In the state in which a raw image is frozen and displayed in the form of a still image, display of a quasi color is enabled. The state then changes to a state in which a still image of a quasi color is, as shown in Fig. 9C, displayed with the raw image kept frozen. If the release switch is handled in this state, the still image of the quasi color is photographed, that is, recorded. Succeeding actions are identical to those performed in the states succeeding the state shown in Fig. 7D.

According to the present embodiment, when a processed image is photographed, a corresponding raw image is displayed in the form of a still image. When a user designates or requires recording, recording can be achieved readily. When the user does not require recording, recording will not be performed. Thus, maneuverability has improved. Moreover, it is avoided to record an image unnecessarily, and it is avoided to take much time to distinguish necessary images from unnecessary ones.

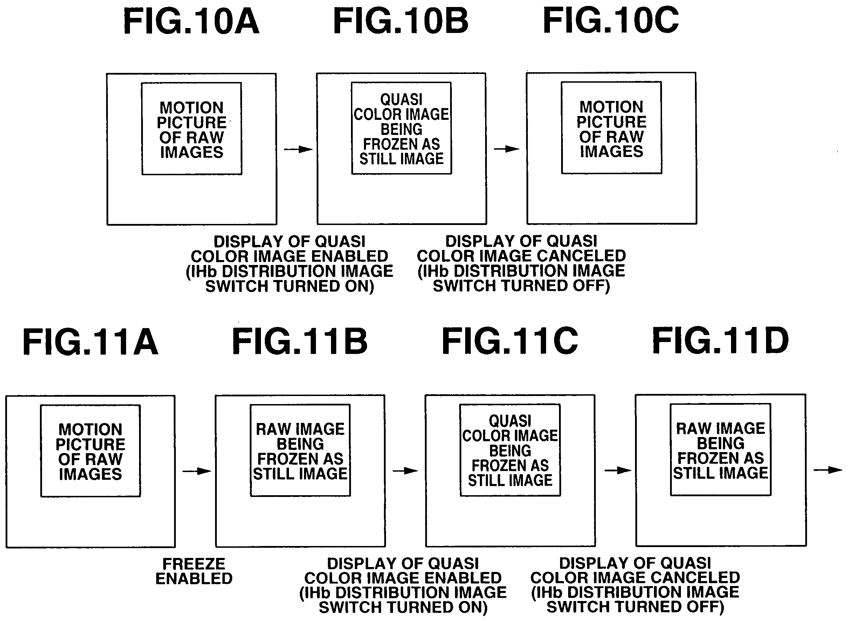

A description has been made of a case where a processed image is exposed. Exposure may not be performed but image processing may be canceled. In this case, if a state attained immediately before a processed image is dealt with is restored, it would be natural to a user. Now, a description will be made in conjunction with Fig. 10A to Fig. 10C that are variants of Fig. 7A to Fig. 7F, and Fig. 11A to Fig. 11D that are variants of Fig. 9A to Fig. 9D.

When a motion picture composed of raw images is displayed as shown in Fig. 10A, if display of a quasi color is enabled, a raw image is frozen as shown in Fig. 10B. A still image of a quasi color is displayed (these actions are identical to those performed in the states indicated in Fig. 7A and Fig. 7B).

Thereafter, when display of a quasi color is disabled, the same state as the state attained before the display of a quasi color is enabled and indicated in Fig. 10A may be restored. Namely, a motion picture composed of raw images may be displayed as shown in Fig. 10C.

Moreover, when a motion picture composed of raw images is displayed as shown in Fig. 11A similarly to the state indicated in Fig. 9A, if freeze is enabled, a raw image is frozen and displayed in the form of a still image as shown in Fig. 11B similarly to Fig. 9B.

Thereafter, if display of a quasi color is enabled, a still image of a quasi color is displayed as shown in Fig. 11C similarly to Fig. 9C. Thereafter, if display of a quasi color is disabled, the same state as the one attained before display of a quasi color is enabled and indicated in Fig. 11B is restored. Namely, a raw image is displayed in the form of a still image as shown in Fig. 11B.

Moreover, if exposure is not performed but image processing is canceled, a state in which a motion picture composed of raw images is displayed may be restored irrespective of the state attained immediately before a processed image is dealt with. In this case, viewing a motion picture can be resumed immediately.

The above description is made on the assumption that an image is recorded by performing photography. The present invention can be adapted to recording of an image using a digital camera or electrical recording of an image in an image filing system or the like.

Moreover, when a quasi color is adopted as a processed image and partly displayed in the center of a raw image display area, if an image may be recorded responsively to the handling of the release switch, a corresponding raw image is consecutively displayed in the form of a still image. A user is permitted to select whether the raw image is recorded or not. If the user wants to record the raw image, only the portion of the raw image corresponding to the partly displayed quasi color may be recorded.

In this case, double recording of the peripheral raw image portion can be prevented. In particular, when an image is electrically recorded, occupation of a memory by an extra image portion can be prevented.

The above description has been made on the assumption that a quasi color image produced based on IHb values is recorded. Even if a processed image is a color-enhanced image or a structure-enhanced image, when the processed image is recorded, a still image of a corresponding raw image may be displayed.

To be more specific, assume that normal image processing (video processing) is performed on an image signal produced by the CCD 28, and color enhancement or structure enhancement is performed on the whole or a predetermined domain of the resultant data of a raw image that can be displayed on the monitor 7. In this case, if the release switch is handled, after an image is recorded by performing photography or the like, a corresponding raw image may be consecutively displayed in the form of a still image.

Moreover, a processed image may be produced by performing a plurality of kinds of image processing on a produced image signal. Even in this case, when the release switch is handled in order to direct exposure of the processed image, after the processed image is recorded, a still image of a corresponding raw image may be displayed.

More particularly, a quasi color image may be displayed as a first processed image in a first predetermined area, for example, in the center of a raw image. A second processed image that is a structure-enhanced or color-enhanced image may be displayed in a second predetermined area around the first predetermined area. If the release switch is handled in order to direct exposure of the compositely processed image, after the processed image is recorded, a corresponding raw image may be displayed in the form of a still image.

Moreover, as far as the compositely processed image is concerned, when a corresponding raw image is displayed in the form of a still image, raw images corresponding to respective processed images may be displayed in the form of still images. Otherwise, a raw image corresponding to one of the processed images may be displayed and the other processed image may be displayed as a still image. Either of the two display modes may be able to be selected.

Moreover, a user may be able to designate the first and the second areas at the keyboard 9 or the like.

Moreover, assume that a plurality of kinds of processing, for example, first processing and second processing may be performed on a user-designated domain of raw image data. In this case, after an image is recorded, a corresponding raw image is displayed in the form of a still image. Otherwise, an image having undergone the first processing and had the second processing canceled may be displayed in the form of a still image. Thereafter, the raw image having the first processing canceled may be displayed in the form of a still image.

Next, a variant of the present embodiment will be described below.

According to the foregoing embodiment, the idea of IHb values serving as hematic information is infused into the concept of image processing. However, other image processing provides a fluorescence, narrow-band, or infrared image. Image processing providing a fluorescence image will be first detailed below.

(1) Fluorescence image

In the configuration shown in Fig. 2, the light source unit 3 field-sequentially irradiates illumination light rays, which fall within regions of wavelengths determining red, green, and blue respectively, to an object to be examined. Component images are produced from the light rays which fall within the regions and with which the object is being illuminated. According to a diagnostic method, a fluorescent substance is administered to an object to be examined in advance, and excitation light that excites the fluorescent substance is irradiated to the object in order to produce a fluorescence image.

A description will be made of points that must be overcome when the diagnostic method is implemented in the aforesaid embodiment. The aforesaid raw image and processed image may be defined in two patterns of pattern A and pattern B as described below.

(A) A raw image is defined as a normal image produced under normal white light (or equivalent field-sequential illumination light having the visible spectrum).
A normal image produced by irradiating white light to an object is regarded as a raw image. For example, a RGB filter that passes visible light is employed.
Moreover, a fluorescence image produced by irradiating excitation light to an object is regarded as a processed image. A filter that passes the infrared spectrum or a filter that limits a spectrum is employed.
(B) A raw image is defined as a fluorescence image.

A fluorescence image produced by irradiating excitation light to an object is regarded as a raw image.

A fluorescent quasi color image produced by performing image processing on a fluorescence image is regarded as a processed image.

According to the method of producing the fluorescent quasi color image, quasi color data is produced within a range of tones employed from a signal which an image pickup device produces based on light received from an object that has become fluorescent. The ratio of the signal, which the image pickup device has produced based on light received from the object that has become fluorescent, to a signal which the image pickup device has produced based on received light reflecting from the object is calculated. Thus, quasi color data may be produced.

Next, a required means and the operation of the means will be described below.

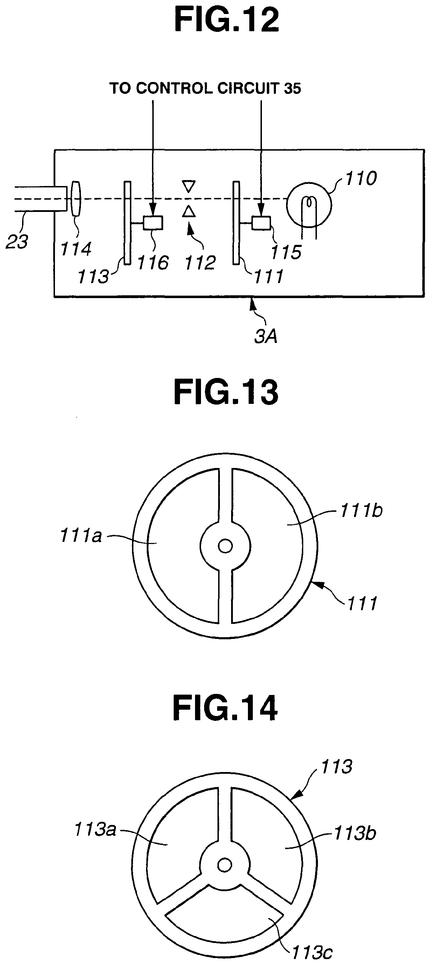

To begin with, a light source unit 3A shown in Fig. 12 is adopted on behalf of the light source unit 3 shown in Fig. 2. The light source unit 3A has the capability of a visible light irradiating means (visible light supplying means) that irradiates (supplies) illumination light, which belongs to the normal visible spectrum, as described below. Moreover, the light source unit 3A has a special light supplying means that supplies special light which belongs to other spectrum, or more particularly, has the ability to irradiate (supply) excitation light used to produce a fluorescence image.

In short, the light source unit 3A has a special light irradiating means that supplies illumination light which belongs to the visible spectrum and any other spectrum alike. When a fluorescence image is produced, a spectrum limiting means limits the illumination light having the visible spectrum.

The video processor 6 performs signal processing so that an image produced under visible illumination light will be provided as a normal image and a fluorescence image will be provided as a processed image.

As shown in Fig. 12, the light source unit 3A comprises: a lamp 110 that radiates light whose wavelengths range from the infrared spectrum to the visible spectrum; a spectrum limiting filter 111 that is located on the path of illumination light emanating from the lamp 110, limits a spectrum to be passed, and can be rotated; an illumination light diaphragm 112 that limits an amount of light emanating from the lamp 110; an RGB rotary filter 113; and a condenser lens 114 that concentrates light.

The spectrum limiting filter 111 and RGB rotary filter 113 are driven to rotate by motors 115 and 116 respectively.

The spectrum limiting filter 111 includes, as shown in Fig. 13, a visible light transmission filter 111a having, for example, a semicircular shape, and an infrared light transmission filter 111b.

The visible light transmission filter 111a or infrared light transmission filter 111b extracts a light component of the light emanating from the lamp 110 which belongs to the visible spectrum or infrared spectrum. The illumination light diaphragm 112 controls the amount of light. The resultant light falls on the RGB rotary filter 113.

The RGB rotary filter 113 has, as shown in Fig. 14, red, green, and blue transmission filters 113a, 113b, and 113c formed circumferentially as if to trisect the RGB rotary filter. When the RGB rotary filter is driven to rotate by the motor 116, the red, green, and blue transmission filters successively intervene in the light path.

Moreover, the light transmission characteristics of the red, green, and blue transmission filters 113a, 113b, and 113c are such that they transmit light rays which belong to regions of wavelengths determining red, green, and blue respectively. Moreover, the red, green, and blue transmission filters 113a, 113b, and 113c pass excitation light whose wavelengths are larger than 700 nm, and transmit light having such wavelengths that excite an indocyanine green (ICG) derivative labeled antibody (for example, infrared light whose wavelengths range from 770 nm to 780 nm).

Light having passed through the RGB rotary filter 113 is concentrated by the condenser lens 114 and irradiated to the incident end of the light guide fiber 23. The light is propagated along the light guide fiber 23, and irradiated from the distal end of the light guide fiber 23 to an object to be examined inside a body cavity through the illumination lens 21.

If the ICG derivative labeled antibody that is a fluorescent substance having an affinity to a lesion such as a tumor is administered to an intracorporeal region of an object to be examined in advance, the fluorescent substance is excited with irradiation of infrared light whose wavelengths range from 770 to 780 nm. This results in fluorescence that causes light of the infrared spectrum ranging from 810 to 820 nm.

Moreover, the motors 115 and 116 are controlled by the control circuit 35.

An excitation light cut filter that is not shown is located in front of the CCD 28 serving as an image pickup means. When fluorescent observation is performed, excitation light is cut in order to pick up the image of feeble light caused by fluorescence. Moreover, a diaphragm that is not shown, has wavelength dependency, and limits an amount of incident light is located on the path of image pickup light linking the objective optical system 22 and CCD 28.

The diaphragm includes, for example, concentric apertures. When visible light passes through the center small circular aperture, it is converged on the CCD 28. In contrast, light caused by fluorescence passes through the center aperture and a surrounding annular aperture and is converged on the CCD 28. Consequently, while a visible light image is formed (with the intensity of the light suppressed), a fluorescence image is formed with the intensity of light held not too small.

According to the present variant, an observation mode selection switch is located on the front panel of the video processor 6. The observation mode selection switch is used to select a first mode in which visible light is utilized or a second mode in which a fluorescence image or a normal endoscopic image is used for observation.

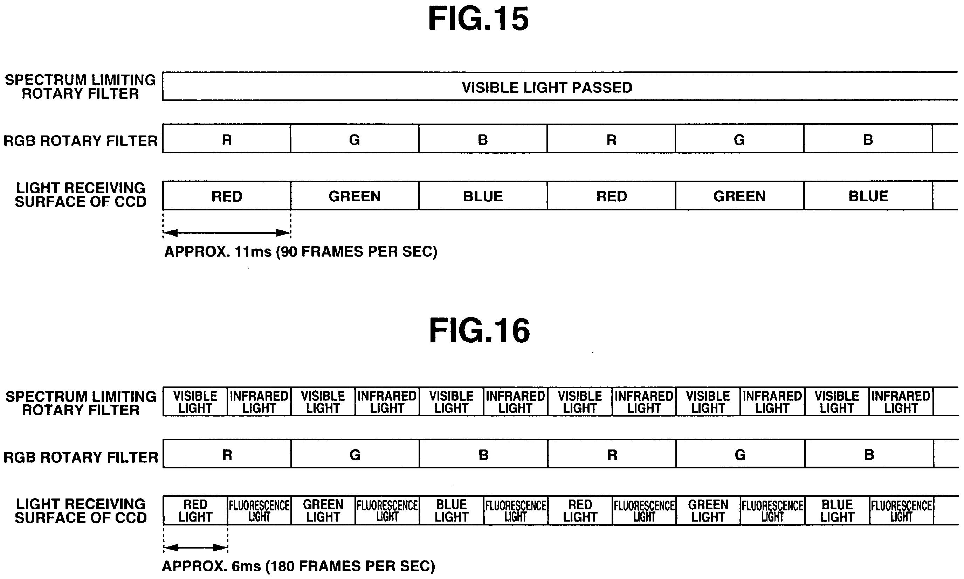

Specifically, when the observation mode selection switch is used to select an observation mode, the directive is transferred to the control circuit 35. The control circuit 35 switches the monitors 115 and 116, and the output terminals of the multiplexer 33 alike. Thus, the control circuit 35 extends control according to each mode in which the states are changed as indicated in Fig. 15 and Fig. 16.

For example, when the first mode identical to the mode implemented in the aforesaid embodiment is selected, the control circuit 35 controls the degree of rotation to which the motor 115 rotates so that the visible light transmission filter 111a included in the spectrum limiting rotary filter 111 will be locked on the light path. Moreover, the control circuit 35 controls the rotation of the motor 116 so that the RGB rotary filter 113 will be rotated 30 revolutions per second.

Fig. 15 shows the states of the activated spectrum limiting rotary filter 111, RGB rotary filter 113, and CCD 28 respectively.

Specifically, in the first mode, the visible light transmission filter 111a included in the spectrum limiting rotary filter 111 is locked on the light path. The RGB rotary filter 113 rotates 30 revolutions per second. Consequently, red, green, and blue light rays are successively irradiated, and images are picked up by the CCD 28. In this case, the same actions as those performed in the aforesaid embodiment are carried out.

In contrast, assume that the second mode is selected (in which visible light is used to form a raw image and a fluorescence image is regarded as a processed image, or a fluorescence image is regarded as a raw image and an image produced by processing the fluorescence-resultant image is regarded as a processed image). In this case, the control circuit 35 controls the rotation of the motor 115 so that the spectrum limiting rotary filter 111 will rotate 90 revolutions per second. Moreover, the control circuit 35 controls the rotation of the motor 116 so that the RGB rotary filter 113 will rotate 30 revolutions per second.

Fig. 16 shows the states of the activated spectrum limiting rotary filter 111, RGB rotary filter 113, and CCD 28 respectively.

At this time, the control circuit 35 extends control so that the RGB rotary filter 113 and spectrum limiting rotary filter 111 will be rotated synchronously with each other.

Specifically, the RGB rotary filter 113 rotates 30 revolutions per second, while the spectrum limiting rotary filter 111 rotates 90 revolutions per second. Consequently, red light, excitation light, green light, excitation light, blue light, and excitation light are successively irradiated.

In this state, while illumination light, that is, red light, infrared light, green light, infrared light, blue light, and infrared light are successively irradiated, the CCD 28 produces red, fluorescence-resultant, green, fluorescence-resultant, blue, and fluorescence-resultant image signals.

A normal image produced under visible light may be adopted as a raw image and a fluorescence image may be adopted as a processed image. Otherwise, a fluorescence image may be adopted as a raw image and a quasi color produced by processing the raw image may be adopted as a processed image. Either of the pairs can be recorded if a user intends so.

For example, as described in as pattern (A), a normal image produced under visible light may be adopted as a raw image, and a fluorescence image may be adopted as a processed image. In this case, after the fluorescence-resultant image that is a processed image is exposed, the normal image that is a raw image is displayed in the form of a still image.

A fluorescence image may be regarded as a raw image, and a fluorescence-resultant quasi color image produced by processing the fluorescence-resultant image may be regarded as a processed image.

A fluorescence quasi color image is produced by quantizing the intensity of fluorescence-resultant light received by the CCD 28. Similarly to a quasi color image based on IHb values, the intensity of received fluorescence-resultant light is divided into signal levels. Quasi colors are assigned to associated signal levels, whereby a fluorescence quasi color image is produced. Otherwise, a ratio of a fluorescence light intensity to the intensity of red or green light received as reflected light may be calculated, and each quasi color may be assigned to the ratio.

Referring to Fig. 12, a single lamp is adopted as a viewing light source means. Alternatively, two or more light sources, such as, a halogen lamp for use in viewing under normal light and a laser for exciting a fluorescent substance or a light-emitting diode may be combined with each other.

(2) Spectrum-limited view image

An endoscope system includes a spectrum limiting means that restricts at least one of a plurality of spectral of illumination light and can provide information concerning the surface of a living tissue at a desired deep region.

In this case, if a spectrum to be passed is not limited, a normal image is produced. The normal image is regarded as a raw image. An image produced with the spectrum to be passed limited is regarded as a processed image. Similarly to a fluorescence image, the processed image that is the image produced with the spectrum to be passed limited is exposed. Thereafter, the raw image is displayed in the form of a still image.

Moreover, the image produced with the spectrum to be passed limited may be regarded as a raw image, and a spectrum-limited quasi color image produced by processing the raw image may be regarded as a processed image. The spectrum-limited quasi color image is produced by quantizing the intensity of light received by the CCD 28 or calculating IHb values, and assigning quasi colors to the resultant signal levels or IHb values according to a range of tones employed.

(3) Infrared light image

An endoscope system permitting viewing of an infrared light image has been disclosed in, for example, Japanese Unexamined Patent Application Publication No. 2000-41942. The infrared light image is regarded as a processed image and a normal image is regarded as a raw image. The idea described in paragraph (1) or (2) can then be adapted.

An infrared image may be regarded as a raw image. The intensity of infrared light received by the CCD 28 may be quantized, or infrared light having a plurality of regions of wavelengths may be employed. The ratio of a signal level produced by the CCD 28 to the intensity of illumination infrared light having each region may be calculated, and each of quasi colors falling within a range of tones employed may be assigned to the ratio. Thus, an infrared quasi color image may be produced.

An amount of indocyanine green (ICG), that is, an ICG value $IIcg$ is calculated as $IIcg=\log(Bin/Rin)$. An infrared quasi color image may be produced based on $IIcg$ values.

Moreover, Japanese Unexamined Patent Application Publication No. 2002-85342 describes production of a quasi color using the distribution of degrees of oxygen saturation caused by infrared light. This quasi color can be dealt with similarly.

(4) 2CCD

A color filter mounted on a CCD may fill the role of limiting a spectrum to be passed. In this case, an electronic endoscope includes both a normal viewing CCD (normal color filter) and a special light viewing CCD (spectrum limiting filter). The special light viewing CCD provides the same signal as the case where an object is illuminated with special light.
An embodiment constructed by mixing parts of the aforesaid embodiment and variant will belong to the present embodiment.
As described so far, according to the present invention, when a user handles the release switch to expose a processed image produced by a processed image producing means, a second still image is recorded responsively to the handling of the release switch. Thereafter, a first still image depicting the same scene as the second still image does is displayed. Thus, a user can select recording or viewing. Thus, the present invention is advantageous because it helps improve maneuverability.

Industrial Applicability

As mentioned previously, when a release switch is handled, an endoscopic image processing system in accordance with the present invention records a second still image of an image whose exposure is directed by handling the release switch. Thereafter, a first still image depicting the same scene as the second still image does is displayed. Thus, either recording or viewing is enabled based on a user's choice. This results in improved maneuverability.

**Claims**

1. An endoscopic image processing system (1) configured to process an image signal produced by imaging an intracavitary region of an object to be examined using an endoscope (2), comprising:

    an image producing means (28) configured to produce a raw image, which can be displayed, by processing the image signal;
    a processed image producing means (41) configured to produce a second endoscopic image, which can be displayed, by processing the image signal or the raw image;
    a still image means configured to produce a raw still image by freezing the image signal or the raw image, and a second still image by freezing the second endoscopic image;
    a memory means (34) in which at least one of the raw and second still images is temporarily stored;
    an image recording means configured to record, in response to a handling of a release switch (10), the raw still image or the second still image; and
    a control means (51) configured to control the endoscopic image processing system (1) to:

        a) display a motion image of the raw image;
        b) display the second still image in the center of the raw image;
        c) in response to a handling of the release switch (10) when the second still image is displayed in the center of the raw image, cause the image recording means to record the second still image;
        d) display the raw still image stored in the memory means (34) after the second still image has been recorded, wherein the raw still image corresponds to the second still image;
        e) after the second still image has been recorded, in response to a directive of exposure activation when the raw still image is displayed, cause the image recording means to record the raw still image corresponding to the recorded second still image; and
        f) after the second still image has been recorded, in response to a freeze canceled directive when the raw still image is displayed, display the motion image of the raw image.

2. The endoscopic image processing system according to Claim 1, wherein the processed image producing means (41) is configured to perform predetermined processing on a predetermined domain within image data representing the raw image so as to produce the second endoscopic image.

3. The endoscopic image processing system according to Claim 2, wherein the processed image producing means (41) is a hematic information calculating means configured to calculate hematic information.

4. The endoscopic image processing system according to Claim 2, wherein the predetermined domain used to produce the second endoscopic image is equal to or smaller than the image data representing the raw image.

5. The endoscopic image processing system according to Claim 1, wherein the processed image producing means (41) includes:

   a special light irradiating means configured to irradiate special light to an object to be examined; and
   a signal processing means (4) configured to perform signal processing on an image signal produced by an image pickup means that is incorporated in the endoscope (2) and image an object to be examined illuminated with the special light.

6. The endoscopic image processing system according to Claim 5, wherein the special light irradiating means includes: an illumination light supplying means (3) configured to supply illumination light having the visible spectrum; and a spectrum limiting means (26) configured to limit at least one of a plurality of spectra of the illumination light.

7. The endoscopic image processing system according to Claim 1, wherein the raw image is an image produced under illumination light having the visible spectrum, or a fluorescence image produced by exciting a region, to which a fluorescent substance has been administered, with excitation light.

8. The endoscopic image processing system according to Claim 1, wherein the processed image producing means (41) is a fluorescence quasi color imaging means configured to produce a quasi color image from a fluorescence image produced by exciting a region, to which a fluorescent substance has been administered, with excitation light.

**Patentansprüche**

1. Endoskopisches Bildverarbeitungssystem (1), das dazu konfiguriert ist, ein Bildsignal zu verarbeiten, das durch Abbilden eines intrakavitären Bereichs eines zu untersuchenden Objekts unter Verwendung eines Endoskops (2) erzeugt wird, umfassend:

   ein Bilderzeugungsmittel (28), das dazu konfiguriert ist, durch Verarbeiten des Bildsignals ein Rohbild zu erzeugen, das angezeigt werden kann;
   ein Erzeugungsmittel für verarbeitete Bilder (41), das dazu konfiguriert ist, durch Verarbeiten des Bildsignals oder des Rohbilds ein zweites endoskopisches Bild zu erzeugen, das angezeigt werden kann;
   ein Standbildmittel, das dazu konfiguriert ist, ein Roh-Standbild durch Einfrieren des Bildsignals oder des Rohbilds und ein zweites Standbild durch Einfrieren des zweiten endoskopischen Bilds zu erzeugen;
   ein Speichermittel (34), in dem mindestens eines von dem Roh-Standbild und dem zweiten Standbild zwischengespeichert wird;
   ein Bildaufzeichnungsmittel, das dazu konfiguriert ist, als Reaktion auf das Bedienen eines Freigabeschalters (10) das Roh-Standbild oder das zweite Standbild aufzuzeichnen; und
   ein Steuerungsmittel (51), das dazu konfiguriert ist, das endoskopische Bildverarbeitungssystem (1) zu steuern, um:

      a) ein bewegtes Bild des Rohbilds anzuzeigen;
      b) das zweite Standbild in der Mitte des Rohbildes anzuzeigen;
      c) als Reaktion auf eine Bedienung des Freigabeschalters (10), wenn das zweite Standbild in der Mitte des Rohbilds angezeigt wird, zu bewirken, dass das Bildaufzeichnungsmittel das zweite Standbild aufzeichnet;
      d) das in dem Speichermittel (34) gespeicherte Roh-Standbild anzuzeigen, nachdem das zweite Standbild aufgezeichnet wurde, wobei das Roh-Standbild dem zweiten Standbild entspricht;
      e) nachdem das zweite Standbild aufgezeichnet wurde, als Reaktion auf eine Anweisung einer Belichtungsaktivierung, wenn das Roh-Standbild angezeigt wird, zu bewirken, dass das Bildaufzeichnungsmittel das dem aufgezeichneten zweiten Standbild entsprechende Roh-Standbild aufzeichnet; und
      f) nachdem das zweite Standbild aufgezeichnet wurde, als Reaktion auf eine Anweisung zum Beenden des Einfrieren, wenn das Roh-Standbild angezeigt wird, das bewegte Bild des Rohbilds anzuzeigen.

2. Endoskopisches Bildverarbeitungssystem nach Anspruch 1, wobei das Erzeugungsmittel für verarbeitete Bilder (41) dazu konfiguriert ist, eine vorbestimmte Verarbeitung an einer vorbestimmten Domäne innerhalb von das Rohbild darstellenden Bilddaten auszuführen, um das zweite endoskopische Bild zu erzeugen.

3. Endoskopisches Bildverarbeitungssystem nach Anspruch 2, wobei das Erzeugungsmittel für verarbeitete Bilder (41) ein Berechnungsmittel für blutbezogene Information ist, das dazu konfiguriert ist, eine blutbezogene Information zu berechnen.

4. Endoskopisches Bildverarbeitungssystem nach Anspruch 2, wobei die zum Erzeugen des zweiten endoskopischen Bildes verwendete vorbestimmte Domäne gleich groß oder kleiner als die das Rohbild darstellenden Bilddaten ist.

5. Endoskopisches Bildverarbeitungssystem nach Anspruch 1, wobei das Erzeugungsmittel für verarbeitete Bilder (41) umfasst:

ein Abstrahlungsmittel für spezielles Licht, das dazu konfiguriert ist, ein spezielles Licht auf das zu untersuchende Objekt zu strahlen; und
ein Signalverarbeitungsmittel (4), das dazu konfiguriert ist, eine Signalverarbeitung an einem Bildsignal durchzuführen, das durch ein in dem Endoskop (2) integriertes Bildaufnahmemittel erzeugt wird, und ein zu untersuchendes Objekt abzubilden, das mit dem speziellen Licht beleuchtet wird.

6. Endoskopisches Bildverarbeitungssystem nach Anspruch 5, wobei das Abstrahlungsmittel für spezielles Licht umfasst: ein Beleuchtungslicht-Zuführmittel (3), das dazu konfiguriert ist, das sichtbare Spektrum aufweisende Beleuchtungslicht zuzuführen; und ein Spektrumsbegrenzungsmittel (26), das dazu konfiguriert ist, mindestens eines von einer Vielzahl von Spektren des Beleuchtungslichts zu begrenzen.

7. Endoskopisches Bildverarbeitungssystem nach Anspruch 1, wobei das Rohbild ein Bild ist, das unter einem das sichtbare Spektrum aufweisende Beleuchtungslicht erzeugt wird, oder ein Fluoreszenzbild ist, das durch Anregen mit Anregungslicht eines Bereichs, dem eine fluoreszierende Substanz zugegeben wurde, erzeugt wird.

8. Endoskopisches Bildverarbeitungssystem nach Anspruch 1, wobei das Erzeugungsmittel für verarbeitete Bilder (41) ein Fluoreszenz-Quasi-Farbabbildungsmittel ist, das dazu konfiguriert ist, ein Quasi-Farbbild aus einem Fluoreszenzbild zu erzeugen, das durch Anregung mit Anregungslicht eines Bereichs, dem eine fluoreszierende Substanz zugegeben wurde, erzeugt wird.

**Revendications**

1. Système de traitement d'image d'endoscope (1) configuré pour traiter un signal d'image produit par imagerie d'une région intracavitaire d'un objet à examiner en utilisant un endoscope (2), comprenant :

un moyen de production d'image (28) configuré pour produire une image brute, qui peut être affichée, en traitant le signal d'image ;
un moyen de production d'image traitée (41) configuré pour produire une seconde image d'endoscope, qui peut être affichée, en traitant le signal d'image ou l'image brute ;
un moyen d'image fixe configuré pour produire une image fixe brute en figeant le signal d'image ou l'image brute, et une seconde image fixe en figeant la seconde image d'endoscope ;
un moyen de mémoire (34) dans lequel au moins une parmi l'image fixe brute et la seconde image fixe est stockée temporairement ;
un moyen d'enregistrement d'image configuré pour enregistrer, en réponse à une manipulation d'un commutateur de libération (10), l'image fixe brute ou la seconde image fixe ; et
un moyen de commande (51) configuré pour commander le système de traitement d'image d'endoscope (1) pour :

a) afficher une image en mouvement de l'image brute ;
b) afficher la seconde image fixe au centre de l'image brute ;
c) en réponse à une manipulation du commutateur de libération (10) lorsque la seconde image fixe est affichée au centre de l'image brute, amener le moyen d'enregistrement d'image à enregistrer la seconde image fixe ;
d) afficher l'image fixe brute stockée dans le moyen de mémoire (34) après que la seconde image fixe a été enregistrée, l'image fixe brute correspondant à la seconde image fixe ;
e) après que la seconde image fixe a été enregistrée, en réponse à une directive d'activation d'exposition lorsque l'image fixe brute est affichée, amener le moyen d'enregistrement d'image à enregistrer l'image fixe brute correspondant à la seconde image fixe enregistrée ; et

f) après que la seconde image fixe a été enregistrée, en réponse à une directive annulée de figeage lorsque l'image fixe brute est affichée, afficher l'image en mouvement de l'image brute.

**2.** Système de traitement d'image d'endoscope selon la revendication 1, dans lequel le moyen de production d'image traitée (41) est configuré pour effectuer un traitement prédéterminé sur un domaine prédéterminé à l'intérieur de données d'image représentant l'image brute de façon à produire la seconde image d'endoscope.

**3.** Système de traitement d'image d'endoscope selon la revendication 2, dans lequel le moyen de production d'image traitée (41) est un moyen de calcul d'informations hématiques configuré pour calculer des informations hématiques.

**4.** Système de traitement d'image d'endoscope selon la revendication 2, dans lequel le domaine prédéterminé utilisé pour produire la seconde image d'endoscope est égal ou plus petit que les données d'image représentant l'image brute.

**5.** Système de traitement d'image d'endoscope selon la revendication 1, dans lequel le moyen de production d'image traitée (41) comprend :

un moyen d'irradiation de lumière spéciale configuré pour émettre une lumière spéciale vers un objet à examiner ; et

un moyen de traitement de signal (4) configuré pour effectuer un traitement de signal sur un signal d'image produit par un moyen de capture d'image qui est incorporé dans l'endoscope (2) et représenter une image d'un objet à examiner éclairé par la lumière spéciale.

**6.** Système de traitement d'image d'endoscope selon la revendication 5, dans lequel le moyen d'irradiation de lumière spéciale comprend : un moyen de distribution de lumière d'éclairage (3) configuré pour distribuer une lumière d'éclairage ayant le spectre visible ; et un moyen de limitation de spectre (26) configuré pour limiter au moins un parmi une pluralité de spectres de la lumière d'éclairage.

**7.** Système de traitement d'image d'endoscope selon la revendication 1, dans lequel l'image brute est une image produite sous une lumière d'éclairage ayant le spectre visible, ou une image de fluorescence produite par excitation d'une région, à laquelle une substance fluorescente a été administrée, avec une lumière d'excitation.

**8.** Système de traitement d'image d'endoscope selon la revendication 1, dans lequel le moyen de production d'image traitée (41) est un moyen d'imagerie de fluorescence en quasi-couleur configuré pour produire une image en quasi-couleur à partir d'une image de fluorescence produite par excitation d'une région, à laquelle une substance fluorescente a été administrée, avec une lumière d'excitation.

# FIG.1

# FIG.2

# FIG.3

# FIG.4A

# FIG.4B

# FIG.4C

# FIG.5

```
┌─────────────────────────────┐
│      PARTIAL DISPLAY OF      │
│      QUASI COLOR IMAGE       │
│  (MASK SIGNAL PRODUCTION)    │
└─────────────────────────────┘
              │
              ▼     S1
         ╱─────────╲          YES
        ╱ CCD=TYPE1? ╲───────────────────────┐
        ╲           ╱                         │        S2
         ╲─────────╱                          ▼  ┌─────────────────────┐
              │ NO                            │  │ PRODUCE MASK SIGNAL │
              │    S3                         └─▶│ ASSOCIATED WITH TYPE1│
              ▼                                  └─────────────────────┘
         ╱─────────╲          YES
        ╱ CCD=TYPE2? ╲───────────────────────┐
        ╲           ╱                         │        S4
         ╲─────────╱                          ▼  ┌─────────────────────┐
              │ NO    S5                         │ PRODUCE MASK SIGNAL │
              ▼                                  │ ASSOCIATED WITH TYPE2│
┌─────────────────────────────┐                 └─────────────────────┘
│     PRODUCE MASK SIGNAL      │
│      ASSOSIATED WITH         │
│         OTHER TYPE           │
└─────────────────────────────┘
```

# FIG.6A

MOTION PICTURE OF RAW IMAGES

# FIG.6B

RAW IMAGE BEING FROZEN AS STILL IMAGE

# FIG.6C

STILL IMAGE OF RAW IMAGE BEING EXPOSED

RECORDING

# FIG.6D

MOTION PICTURE OF RAW IMAGES

FREEZE ENABLED

EXPOSURE ENABLED

# FIG.9A

MOTION PICTURE OF RAW IMAGES

# FIG.9B

RAW IMAGE BEING FROZEN AS STILL IMAGE

# FIG.9C

QUASI COLOR IMAGE BEING FROZEN AS STILL IMAGE

# FIG.9D

STILL IMAGE OF QUASI COLOR IMAGE BEING EXPOSED

RECORDING

FREEZE ENABLED

DISPLAY OF QUASI COLOR IMAGE ENABLED (IHb DISTRIBUTION IMAGE SWITCH TURNED ON)

EXPOSURE ENABLED

EP 1 484 001 B1

# FIG.7A
MOTION PICTURE OF RAW IMAGES

# FIG.7B
QUASI COLOR IMAGE BEING FROZEN AS STILL IMAGE

# FIG.7C
STILL IMAGE OF QUASI COLOR IMAGE BEING EXPOSED

RECORDING

DISPLAY OF QUASI COLOR IMAGE ENABLED (IHb DISTRIBUTION IMAGE SWITCH TURNED ON)

EXPOSURE ENABLED

# FIG.7D
RAW IMAGE BEING FROZEN AS STILL IMAGE

FREEZE CANCELED

# FIG.7E
STILL IMAGE OF RAW IMAGE BEING EXPOSED

RECORDING

# FIG.7F
MOTION PICTURE OF RAW IMAGES

EXPOSURE ENABLED

EP 1 484 001 B1

# FIG.8

```
                    ( START )
S11
        DISPLAY RAW IMAGES
        IN FORM OF MOTION PICTURE

S12
        < EXPOSURE=ON? > ──NO──┐
                │ YES          │
S13                            │
        < DISPLAY OF ──NO──┐   │
        QUASI COLOR IMAGE? │   │
                │ YES      │   │
S15                    S14 ▼   │
  PHOTOGRAPH PROCESSED   PHOTOGRAPH RAW IMAGE
        IMAGE             │
                │ ◄───────┘
S16
  FREEZE RAW IMAGE
  TO PRODUCE STILL IMAGE

S17
        < FREEZE=ON? > ──NO──┐
                │ YES        │
S18                          │
  NO── < EXPOSURE=ON? >      │
                │ YES        │
S19                          │
  PHOTOGRAPH RAW IMAGE       │
                │ ◄──────────┘
S20
  DISPLAY MOTION PICTURE
  OF RAW IMAGES
```

EP 1 484 001 B1

FIG.10A — MOTION PICTURE OF RAW IMAGES

DISPLAY OF QUASI COLOR IMAGE ENABLED (IHb DISTRIBUTION IMAGE SWITCH TURNED ON)

FIG.10B — QUASI COLOR IMAGE BEING FROZEN AS STILL IMAGE

DISPLAY OF QUASI COLOR IMAGE CANCELED (IHb DISTRIBUTION IMAGE SWITCH TURNED OFF)

FIG.10C — MOTION PICTURE OF RAW IMAGES

FIG.11A — MOTION PICTURE OF RAW IMAGES

FREEZE ENABLED

FIG.11B — RAW IMAGE BEING FROZEN AS STILL IMAGE

DISPLAY OF QUASI COLOR IMAGE ENABLED (IHb DISTRIBUTION IMAGE SWITCH TURNED ON)

FIG.11C — QUASI COLOR IMAGE BEING FROZEN AS STILL IMAGE

DISPLAY OF QUASI COLOR IMAGE CANCELED (IHb DISTRIBUTION IMAGE SWITCH TURNED OFF)

FIG.11D — RAW IMAGE BEING FROZEN AS STILL IMAGE

26

# FIG.12

TO CONTROL CIRCUIT 35

110

23 114 113 116 112 115 111

3A

# FIG.13

111a 111b

111

# FIG.14

113

113a 113b

113c

# FIG.15

| | | | | | | |
|---|---|---|---|---|---|---|
| SPECTRUM LIMITING ROTARY FILTER | VISIBLE LIGHT PASSED | | | | | |

| | | | | | | |
|---|---|---|---|---|---|---|
| RGB ROTARY FILTER | R | G | B | R | G | B |

| | | | | | | |
|---|---|---|---|---|---|---|
| LIGHT RECEIVING SURFACE OF CCD | RED | GREEN | BLUE | RED | GREEN | BLUE |

APPROX. 11ms (90 FRAMES PER SEC)

# FIG.16

| | | | | | | | | | | | | |
|---|---|---|---|---|---|---|---|---|---|---|---|---|
| SPECTRUM LIMITING ROTARY FILTER | VISIBLE LIGHT | INFRARED LIGHT | VISIBLE LIGHT | INFRARED LIGHT | VISIBLE LIGHT | INFRARED LIGHT | VISIBLE LIGHT | INFRARED LIGHT | VISIBLE LIGHT | INFRARED LIGHT | VISIBLE LIGHT | INFRARED LIGHT |

| | | | | | |
|---|---|---|---|---|---|
| RGB ROTARY FILTER | R | G | B | R | G | B |

| | | | | | | | | | | | | |
|---|---|---|---|---|---|---|---|---|---|---|---|---|
| LIGHT RECEIVING SURFACE OF CCD | RED LIGHT | FLUORESCENCE LIGHT | GREEN LIGHT | FLUORESCENCE LIGHT | BLUE LIGHT | FLUORESCENCE LIGHT | RED LIGHT | FLUORESCENCE LIGHT | GREEN LIGHT | FLUORESCENCE LIGHT | BLUE LIGHT | FLUORESCENCE LIGHT |

APPROX. 6ms (180 FRAMES PER SEC)

EP 1 484 001 B1

**EP 1 484 001 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2851116 B
- US 5045935 A
- US 4712133 A

- JP 2000041942 A
- JP 2002085342 A